# EUROPEAN PATENT APPLICATION

(11) **EP 4 840 480 A1**
(43) Date of publication of application: **18.11.2026**
(21) Application number: 25739096.3
(22) Date of filing: 09.01.2025
(51) Int. Cl.: A61L 27/60, A61K 35/36, A61P 17/02, A61L 26/00, A61L 27/36

(54) **COMPOSITION COMPRISING FIBROUS ACELLULAR DERMAL MATRIX PREPARED BY DRYING, AND METHOD FOR PREPARING SAME**

(30) Priority: 10.01.2024 KR 20240003879; 02.02.2024 KR 20240016543; 08.01.2025 KR 20250003025
(71) Applicant: CG Bio Co., Ltd., Seoul 04791 (KR)
(72) Inventor: WOO, Jiwon, Bucheon-si Gyeonggi-do 14417 (KR); LEE, Kwon Young, Seongnam-si Gyeonggi-do 13172 (KR); YOON, Hong Sun, Yongin-si Gyeonggi-do 17117 (KR)
(74) Representative: Viering, Jentschura & Partner mbB Patent- und Rechtsanwälte
(86) International application number: PCT/KR2025/000520
(87) International publication number: WO 2025/150931

(57) **Abstract**

The present invention relates to: a composition comprising a fibrous acellular dermal matrix, which does not employ a biocompatible polymer and is subjected to a drying process using a sieve, thereby exhibiting improved physical properties, tensile strength, and suture strength, and thus providing enhanced usability compared to conventional compositions; and a method for preparing same.

## Description

### Technical Field

The present disclosure relates to a composition comprising a fibrous acellular dermal matrix and a method for preparing the same, and more particularly, to a composition comprising a drying method-applied fibrous acellular dermal matrix and a method for preparing the same.

### Background Art

A wound is a condition in which the continuity of the tissue is destroyed by the rupture or defect of the skin or other tissue due to external pressure, and usually refers to a phenomenon in which the skin is opened by damaging the dermis layer of the skin. Wound surgery basically prevents infection and suppresses inflammatory reactions by suturing and dressing the wound site to block it from being exposed to the external environment. The materials for wound dressings can be largely divided into allogeneic dermis and xenogeneic dermis, and the wound dressings can be manufactured by a method of extracting specific polymers from the materials and a method of using a dermal matrix.

Acellular dermal matrix (ADM) is one in which the cells in the dermis are removed after removing the epidermis from the skin tissue of a donated cadaver in order to eliminate immune rejection reactions, and it is widely used in soft tissue reconstruction in this field and is also widely used in allograft for burn treatment. The dermal tissue is composed of 80 to 90% collagen, elastin, and glycosaminoglycan.

Meanwhile, in 1994, LifeCell prepared an acellular dermal matrix (ADM) by decellularizing and freeze-drying skin tissue collected from cadavers and commercialized it as Alloderm (allograft) for use in burn treatment, skin reconstruction, etc. Alloderm (allograft) was safer than heterogeneous products and showed much better survival rate and healing effect. As similar products, Bard Dabol's AlloMax, Ethicon's FlexHD, etc. were developed. However, the products described as above had the problem of having poor flexibility, making them difficult to use on curved or deep wounded areas.

To solve such a problem, LifeCell developed a technology to micronize acellular dermal matrix in 1999 and launched an AlloDerm (Cymetra) product in the form of an injectable micronized preparation. In addition, Wright Medical Group developed the Graftjacket product in the form of an injectable preparation and launched the Graftjacket Xpress product, and LNC Bio developed a composition in which hyaluronic acid was cross-linked after micronizing acellular dermal matrix in 2014. However, in the case of the composition in which acellular dermal matrix particles and biocompatible polymers as described above were cross-linked, it had high structural stability, but also had high cross-linking degree, viscoelasticity, hardening degree, and extrusion force, making it suitable for use as fillers and prosthetics, but it was still insufficient for application as a wound dressing. In the case of the micronized form, there was a problem that it could not be fixed to the wound site in a liquid form and flowed out of the dressing, preventing it from being evenly distributed on the wound.

Therefore, there is an urgent need to develop a form of an acellular dermal matrix that can be fixed to the wound site.

### Disclosure

### Technical Problem

An object of the present disclosure, which has been derived to solve the problems described above, is to provide a composition including a drying method-applied fibrous acellular dermal matrix which is easy to apply to curved wound sites and has enhanced physical properties by applying a process of drying in a sieve without applying a biocompatible polymer, and a method for manufacturing the same.

### Technical Solution

In order to achieve the above object, a composition comprising a drying method-applied fibrous acellular dermal matrix according to one embodiment of the present disclosure includes 48 to 95.5% by weight of a fibrous acellular dermal matrix and 0.5 to 52% by weight of water, wherein the composition has no biocompatible polymer added thereto, and the fibrous acellular dermal matrix includes an antibacterial agent at a concentration of 1 to 1,000,000 U/mL.

A method for preparing a composition comprising a drying method-applied fibrous acellular dermal matrix according to another embodiment of the present disclosure includes steps of (a) pulverizing an acellular dermal matrix to prepare a fibrous acellular dermal matrix, (b) adding water to the fibrous acellular dermal matrix to rehydrate it, and (c) heat-drying the rehydrated fibrous acellular dermal matrix.

### Advantageous Effects

According to one embodiment of the present disclosure, since a composition including a drying method-applied fibrous acellular dermal matrix and a method for preparing the same do not have a biocompatible polymer added thereto, there is no change in physical properties due to heat, and a step for heat-treating a biocompatible polymer is not required, so the preparation process can be simplified and the preparation cost can be reduced.

In addition, since it has a structure very similar to the dermal matrix of a wound site of a human body, it has excellent bioadhesiveness and preservability in the body, and since a moist environment is well maintained, the treatment effect is excellent and it can be easily applied to a curved wound site.

In addition, by performing a step of drying in a sieve, physical properties, tensile strength, and suture strength are improved, so that the operator can use it more easily during the procedure, and it can also serve as a physical barrier and be used as an anti-adhesion agent.

### Description of Drawings

FIG. 1 is a process diagram for explaining the method for preparing a composition including a drying method-applied fibrous acellular dermal matrix according to another embodiment of the present disclosure.
FIGS. 2 and 3 are graphs showing the results according to Experimental Example 1.
FIG. 4 is a photograph showing the results according to Experimental Example 2.

### Mode for Disclosure

Hereinafter, the present disclosure will be described in detail with reference to the attached drawings as embodiments of the present disclosure. However, the following embodiments are presented as examples of the present disclosure, and if it is judged that a specific description of a well-known technology or configuration to those skilled in the art may unnecessarily obscure the gist of the present disclosure, the detailed description thereof may be omitted, and the present disclosure is not limited thereby. The present disclosure can be variously modified and applied within the description of the patent claims described later and the equivalent scope interpreted therefrom.

In addition, the terminologies used in this specification are terminologies used to appropriately express the preferred embodiments of the present disclosure, and these may vary depending on the intention of the user or operator or the customs of the field to which the present disclosure belongs. Therefore, the definition of these terminologies should be made based on the contents throughout this specification. Throughout the specification, when a part is said to "include" a certain component, this does not mean that, unless otherwise specifically stated, other components are excluded, but other components may be further included.

Throughout this specification, '%' used to indicate the concentration of a specific substance, unless otherwise stated, is %(w/w) for solid/solid, %(w/v) for solid/liquid, and %(v/v) for liquid/liquid.

Hereinafter, a composition including a fibrous acellular dermal matrix according to one embodiment of the present disclosure will be described in detail.

The composition including a fibrous acellular dermal matrix according to the present embodiment includes a fibrous acellular dermal matrix and water, wherein the fibrous acellular dermal matrix includes an antibacterial agent.

Acellular dermal matrix (ADM) is a component widely used for reconstruction, regeneration, and strengthening of skin, tendons, and ligaments in plastic surgery and orthopedics due to its excellent biocompatibility with high cell adhesion ability and low immune response compared to conventional animal-derived products. The fibrous acellular dermal matrix used in this embodiment may include 90% or more of those having a major axis length of 10 to 3,000 µm, preferably 100 to 2,000 µm, and more preferably 50 to 90% of those having a major axis length of 200 to 800 µm. This is because if a large number of particles less than 100 µm are included, the shape may not be properly formed when processing into a sheet type, or it may be made only in a film form, making it difficult to flexibly adhere to the wound site. Such a fibrous acellular dermal matrix may be included in an amount of 48 to 95.5% by weight.

Meanwhile, the fibrous acellular dermal matrix may include an antibacterial agent, and the antibacterial agent may be included at a concentration of 1 to 1,000,000 U/mL, for example, 300,000 to 700,000 U/mL. If the concentration of the antibacterial agent is less than 1 U/mL, it may be difficult to implement an antibacterial effect of Log 5 or more, and if it exceeds 1,000,000 U/mL, the risk of toxicity may increase, and the effect of using more than necessary may not be very significant.

In this embodiment, the antibacterial agent is a component added to impart antibacterial properties, and at least one selected from Polymyxin B, Vancomycin, Amphotericin B, short-chain alcohols, benzalkonium chloride (BAC), didecyl dimethyl ammonium chloride (DDAC), zeolite (CWT-A), isothiazolone, alkyl dimethyl ammonium chloride, triazine, 2-thiocyanomethylthio benzothiazole, methylenebis thiocyanate, acrolein, dodecylguanidine hydrochloride, chlorophenol, quaternary ammonium salts, glutaraldehyde, dithiocarbamate, 2-mercaptobenzothiazole, para-chloro-meta-xylenol, silver, chlorhexidine, polyhexamethylene biguanide, n-halamine, triclosan, phospholipids, alpha hydroxy acids, 2,2-dibromo-3-nitrilopropionamide, 2-bromo-2-nitro-1,3-propanediol, farnesol, iodine, bromine, hydrogen peroxide, chlorine dioxide, vegetable oils, plant extracts, chlorine, and sodium hypochlorite may be used. For example, at least one selected from Polymyxin B, Vancomycin, and Amphotericin B can be used, and when the above-mentioned conditions are satisfied, a high antibacterial effect of Log 5 or more can be exhibited.

Water is a component added for rehydration and may be included in an amount of 0.5 to 52% by weight. If water is less than 0.5% by weight, it may be difficult to hydrate the fibrous acellular dermal matrix, and if it exceeds 52% by weight, it may be difficult to implement the desired formulation due to use more than necessary.

In this embodiment, no biocompatible polymer is added, so there is no change in physical properties due to heat, and a step for heat-treating the biocompatible polymer is not required, thereby simplifying the manufacturing process and reducing manufacturing costs. In addition, since it has a structure very similar to the dermal matrix of the wound site of the human body, it has excellent bioadhesiveness and preservability in the body, and the moist environment is well maintained, so the treatment effect is excellent and it can be easily applied to a curved wound site.

In the present embodiment, the term fibrous acellular dermal matrix may mean that the individual form of the particleized acellular dermal matrix is a thin and long fiber shape like a thread, rather than a spherical or streamlined shape.

The present embodiment may further include stem cells, growth factors, or a mixture thereof.

In addition, the present embodiment may further include one or more selected from pharmaceutically used excipients and additives.

As excipients, one or more selected from stabilizers, antioxidants, osmotic pressure-adjusting agents, buffers, and pH-adjusting agents can be used. Specifically, one or more selected from starch, cellulose, glucose, lactose, sucrose, gelatin, corn, rice, flour, chalk, silica gel, magnesium stearate, sodium stearate, glycerol monostearate, sodium chloride, glycerol, propylene glycol, water, and ethanol can be used.

As additives, one or more selected from physiologically biocompatible buffers (tromethamine hydrochloride), chelating agents (DTPA or DTPA-bisamide), and calcium chelate complexes (calcium DTPA, CaNaDTPA-bisamide) can be used, and optionally, calcium or sodium salts (calcium chloride, calcium ascorbate, calcium gluconate, or calcium lactate) can be used.

The composition may further include one or more of stem cells and growth factors.

In the present embodiment, the composition comprising the fibrous acellular dermal matrix may be prepared in the form of an aqueous solution, suspension, emulsion, paste, cream, balm, ointment, foam, sheet, gel, gum, spray, slurry, film, granule, patch, powder, and the like, but may preferably be a sheet type, and can be used as a dressing, adhesive, surgical and medical device, artificial skin, bandage, foaming agent, anti-adhesion agent, or implant.

Hereinafter, a method for preparing a composition comprising a fibrous acellular dermal matrix according to another embodiment of the present disclosure will be described in detail with reference to the drawings.

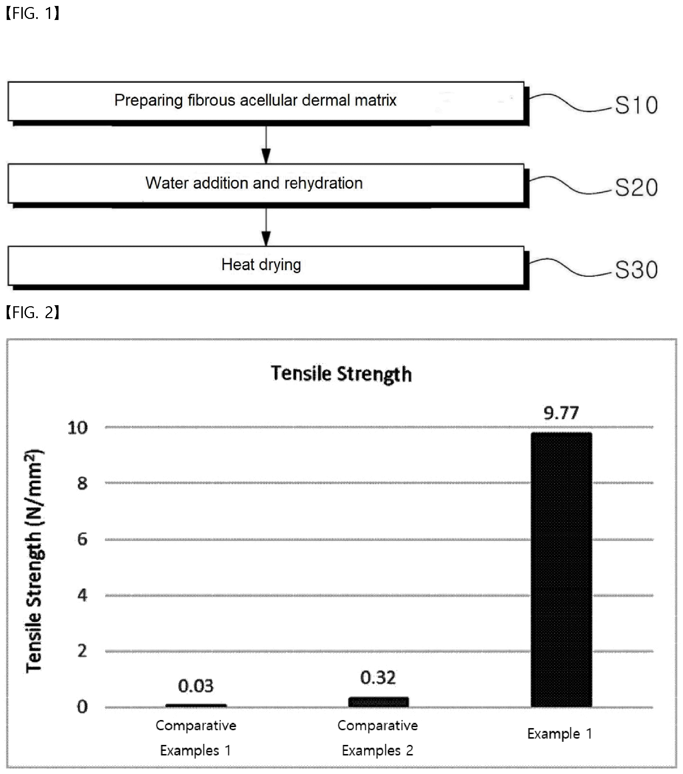

FIG. 1 is a process diagram for explaining a method for preparing a composition comprising a fibrous acellular dermal matrix according to another embodiment of the present disclosure.

Referring to FIG. 1, first, an antibacterial agent is added to and infiltrated into an acellular dermal matrix, followed by freeze-drying and pulverization to prepare a fibrous acellular dermal matrix (S10).

The fibrous acellular dermal matrix may be prepared by adding an antibacterial agent at a concentration of 1 to 1,000,000 U/mL, for example, 300,000 to 700,000 U/mL, to the acellular dermal matrix, infiltrating the same in a low-temperature reactor, freeze-drying for 20 to 80 hours, and then pulverizing with at least one pulverizer selected from a cutting mill, a food processor, an agate grinder, a cryogenic pulverizer, a micronizer, a vibratory micro mill, a jaw crusher, a mortar grinder, a planetary mill, a disk mill, a ball mill, a knife mill, and a variable speed rotor mill. At this time, it is preferable to set the pulverizer at a rotation speed of 500 to 2,000 rpm to facilitate pulverization in a short period of time.

Meanwhile, in the present embodiment, the acellular dermal matrix may be one prepared by steps of: preparing human-derived skin tissue that has not undergone separate deepithelialization and defatting processes; treating the skin tissue with a hypotonic solution containing a surfactant; and washing the treated skin tissue with an isotonic solution. Here, it is preferable to apply an acellular dermal matrix satisfying a thickness of 0.1 mm to 3 mm.

Next, water is added to the fibrous acellular dermal matrix to rehydrate it (S20).

The fibrous acellular dermal matrix prepared in step S10 may be hydrated by adding 300 to 2,000 parts by weight of water to 100 parts by weight of the fibrous acellular dermal matrix. If the water content is less than 300 parts by weight, it may be difficult to hydrate the fibrous acellular dermal matrix, and if it exceeds 2,000 parts by weight, it is not preferable because it may prolong the heat-drying time, which will be described later, due to use more than necessary. In the present embodiment, the water may be sterile distilled water.

Then, the rehydrated fibrous acellular dermal matrix is heat-dried (S30).

The fibrous acellular dermal matrix rehydrated in step S20 may be heat-dried in a sieve, preferably a sieve having a mesh size of 0.0001 to 5 mm, at 30 to 50°C for 6 hours or more, for example, 10 hours or more, for example, 10 to 24 hours, preferably 16 to 18 hours, until the water content reaches 0.5 to 52% by weight. If the temperature is less than 30°C, it may take excessive time to dry, and if it exceeds 50°C, the human tissue may be deformed, leading to a decrease in amino acid content as well as a decrease in tensile strength and hydration degree.

Under the heat-drying conditions as described above, the maximum tensile strength value of the fibrous acellular dermal matrix is 9.7 N/mm² or more and the maximum suture strength value is 7.3 N/mm² or more, so that the operator can use it more easily during the procedure compared to conventional ones. In addition, by drying through a sieve, the structure is dense and it can also serve as a physical barrier.

After step S30, a step of processing the heat-dried fibrous acellular dermal matrix into a sheet type may be further included.

The fibrous acellular dermal matrix heat-dried in step S30 may be applied to a mold or a frame and then shaped and processed into a sheet type, and it goes without saying that it can be manufactured in various ways according to the width, length, and thickness sizes required by the wound site or surgical characteristics.

Meanwhile, prior to processing into a sheet type, a non-chemical cross-linking process using irradiation, reduced pressure, and heat, or a chemical cross-linking process using a cross-linking agent, may be additionally performed depending on physical properties (cross-linking degree, hardening degree, etc.).

Hereinafter, the present disclosure will be described in more detail using examples. These examples are only for explaining the present disclosure in more detail, and it is obvious to those of ordinary skill in the art that the scope of the present disclosure is not limited by them.

### Preparation Example 1. Preparation of Acellular Dermal Matrix

Skin tissues were purchased from EURO skin bank, Allosource, and CTS, and tissues having a thickness of 0.5 mm or more were selected. Fat tissue attached to the skin tissue was removed using forceps, and then washed three times with sterile water. Next, it was immersed in a hypotonic solution (Tris-HCl, EDTA, NaOH, and SDS) and treated for 6 hours. Then, the skin tissue was washed with PBS at 4°C to remove residual fat, epidermis, cells, and the hypotonic solution from the skin tissue, and after an overnight period, it was washed with an isotonic solution (Tris-HCl, EDTA, NaCl, NaOH) for 6 hours to prepare a deepithelialized, defatted, and decellularized human-derived acellular dermal matrix.

### Example 1. Preparation of Composition Comprising Fibrous Acellular Dermal matrix

A 14.3% maltitol solution and an antibacterial agent at a concentration of 1,000,000 U/mL were added to the human-derived acellular dermal matrix prepared in Preparation Example 1, followed by infiltration in a low-temperature reactor at 4°C. After placing the same in Tyvek (Koreacns, Korea), it was dried in a freeze-dryer at a vacuum of 5 torr for 24 hours and pulverized with a cutting mill to be fibrous, thereby preparing a fibrous acellular dermal matrix. Ten times the amount of sterile distilled water was added thereto to rehydrate it, placed on a sieve having a size of 100 µm, heat-dried at 30°C to 50°C for 6 hours or more, applied to a mold to shape it, and processed into a sheet type to prepare a composition comprising a fibrous acellular dermal matrix.

### Comparative Example 1. Preparation of Composition Comprising Fibrous Acellular Dermal Matrix

A composition was prepared in the same manner as in Example 1, except that freeze-drying was performed instead of heat-drying.

### Comparative Example 2. Preparation of Composition Comprising Fibrous Acellular Dermal Matrix

A composition was prepared in the same manner as in Example 1, except that heat-drying was performed in a metal mold instead of using a sieve.

### Experimental Example 1: Evaluation of Physical Properties

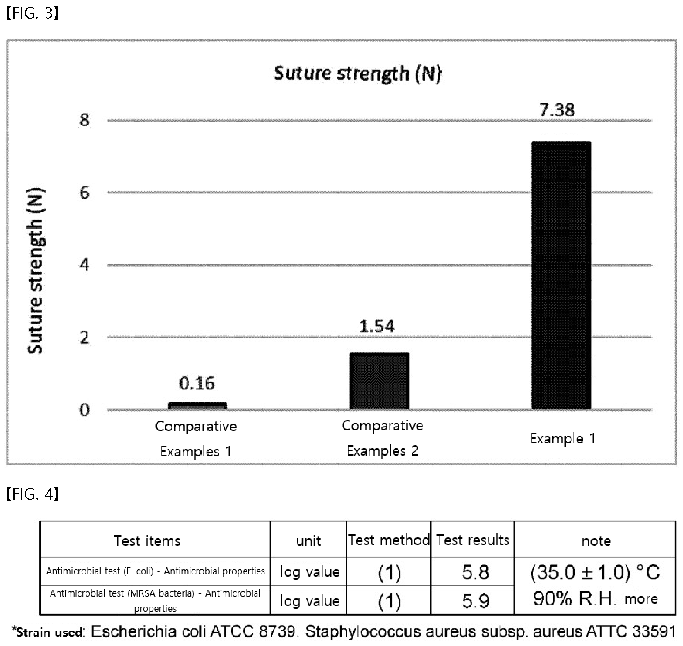

To understand the physical properties of the present disclosure, the tensile strength and suture strength of Example 1 and Comparative Examples 1 and 2 were measured, and the results are shown in FIGS. 2 and 3.

Referring to FIGS. 2 and 3, it was confirmed that the tensile strength and suture strength of Comparative Examples 1 and 2 were far below those of Example 1.

This indicates that the composition comprising the fibrous acellular dermal matrix prepared according to Example 1 of the present disclosure has a dense structure and improved physical properties, tensile strength, and suture strength by performing the heat-drying, particularly the heat-drying in a sieve, thereby making it easier for an operator to use during a procedure.

### Experimental Example 2: Evaluation of Antibacterial Performance

An experiment was conducted based on JIS Z 2801:2012 to confirm the presence or absence of antibacterial activity of the present disclosure, and the results are shown in FIG. 4.

The bacterial strains used for the evaluation of antibacterial performance were Escherichia coli (ATCC 8739) and Staphylococcus aureus subsp. aureus (ATCC 33591) obtained from ATCC.

Referring to FIG. 4, Example 1 showed antibacterial performance with a value of Log 5 or more.

Therefore, it was confirmed that the composition comprising the fibrous acellular dermal matrix prepared according to Example 1 of the present disclosure has excellent antibacterial and antimicrobial performance.

While the exemplary embodiments of the present disclosure have been described in detail above, the scope of rights of the present disclosure is not limited thereto, and various modifications and improvements by those skilled in the art using the basic concept of the present disclosure defined in the following claims also fall within the scope of rights of the present disclosure.

All technical terms used in the present disclosure have the same meanings as commonly understood by those of ordinary skill in the related field of the present disclosure, unless otherwise defined. The contents of all publications described as references in this specification are incorporated into the present disclosure.

## Claims

1. A composition comprising a fibrous acellular dermal matrix, the composition comprising 48 to 95.5% by weight of the fibrous acellular dermal matrix and 0.5 to 52% by weight of water, wherein no biocompatible polymer is added thereto, and the fibrous acellular dermal matrix includes an antibacterial agent at a concentration of 1 to 1,000,000 U/mL.

2. The composition according to claim 1, wherein the composition satisfies a maximum tensile strength value of 9.7 N/mm² or more and a maximum suture strength value of 7.3 N/mm² or more.

3. The composition according to claim 1, further comprising stem cells, growth factors, or a mixture thereof.

4. The composition according to claim 1, wherein the composition is a sheet type.

5. A method for preparing a composition comprising a fibrous acellular dermal matrix, the method comprising steps of: (a) preparing a fibrous acellular dermal matrix by adding an antibacterial agent to an acellular dermal matrix for infiltration, followed by freeze-drying and pulverization; (b) adding water to the fibrous acellular dermal matrix to rehydrate it; and (c) heat-drying the rehydrated fibrous acellular dermal matrix.

6. The method according to claim 5, wherein the heat-drying in step (c) is performed at 30 to 50°C for 6 hours or more.

7. The method according to claim 5, wherein the heat-drying in step (c) is performed in a sieve.

8. The method according to claim 5, further comprising a step (d) of processing the heat-dried fibrous acellular dermal matrix into a sheet type after step (c).
